# EUROPÄISCHE PATENTANMELDUNG

(11) **EP 3 747 394 A1**
(43) Veröffentlichungstag der Anmeldung: **09.12.2020**
(21) Anmeldenummer: 19178173.1
(22) Anmeldetag: 04.06.2019
(51) Int. Cl.: A61C 8/00, A61F 2/00, A61L 27/00

(54) **ZAHNIMPLANTAT MIT UNTERSCHIEDLICHEN OBERFLÄCHENRAUHEITEN**

(71) Anmelder: VITA-ZAHNFABRIK H. Rauter GmbH & Co. KG, 79713 Bad Säckingen (DE)
(72) Erfinder: SCHÖNE, André, 17498 Behrenhoff OT Stresow (DE)
(74) Vertreter: dompatent von Kreisler Selting Werner - Partnerschaft von Patent- und Rechtsanwälten mbB

(57) **Zusammenfassung**

Die vorliegende Erfindung betrifft ein Zahnimplantat, das verschiedene Bereiche mit unterschiedlichen Oberflächenrauheiten aufweist.

## Beschreibung

Die vorliegende Erfindung betrifft ein Zahnimplantat, das verschiedene Bereiche mit unterschiedlichen Oberflächenrauheiten aufweist.

Implantate sind in den Körper eingepflanzte künstliche Materialien, die permanent oder zumindest für einen längeren Zeitraum im Körper verbleiben sollen. Zahnimplantate werden in den Kieferknochen eingesetzt und übernehmen dort als Träger von prothetischen Versorgungen die Funktion einer künstlichen Zahnwurzel. Hierzu werden sie entweder mittels Schraubgewinde in den Kieferknochen eingedreht oder eingesteckt. Das Ziel ist es, dass sich das Implantat mit dem umgebenen Knochen zu einer festen, äußerst belastbaren Trägereinheit verbindet. Die Verwendung solcher Zahnimplantate hat den Vorteil, dass für die Befestigung der prothetischen Versorgung, wie Brücken oder Einzelkronen, nicht auf die angrenzenden Zähne zurückgriffen werden muss, so dass diese nicht beschliffen werden müssen.

Von den Zahnimplantaten wird dabei erwartet, dass sie zum einen eine stabile Stütze für den auf ihnen angebrachten Zahnersatz bieten und zum anderen eine schnelle und dauerhafte Verbindung mit dem sie umgebenden Gewebe eingehen. Um beispielsweise das Einwachsen des Zahnimplantats zu fördern, hat es sich als vorteilhaft erwiesen, das Implantat mit einer angerauten Oberfläche zu versehen, die die Anlagerung von Knochenzellen erleichtert. Demgegenüber steht die Forderung nach einer Oberfläche, auf der die prothetische Versorgung aufgebracht und verbunden werden kann.

EP 0 388 576 beschreibt ein mit einer porösen Oberfläche versehenes, zum Einsetzen in einen Knochen bestimmtes Implantat, dessen Oberfläche eine Mikrorauigkeit mit einer Porengröße in der Größenordnung von 2 µm und weniger aufweist.

DE 10 2005 006 979 offenbart ein enossales Zahnimplantat mit einem Implantatkörper, der für das Einsetzen in einen Kieferknochen ausgebildet ist, und einem Implantatpfosten, der einen Anschlussabschnitt zum Anschluss eines prothetischen Aufbaus aufweist, wobei zumindest der Implantatkörper aus einem keramischen Material hergestellt ist und wobei das Implantat mindestens zweiteilig ausgebildet ist, so dass Implantatkörper und Implantatpfosten getrennte Teile sind.

EP 1 982 671 offenbart ein Zahnimplantat mit einer Oberfläche aus einem keramischen Material, die eine Topographie mit einer Kernrauhtiefe Sₖ von kleiner als 1 µm und eine Schiefe Sₛₖ von kleiner 0 aufweist.

Obwohl der Stand der Technik eine Reihe von Lösungen bereitstellt, wie das Einwachsen des Implantats in den Knochen gefördert werden kann, besteht weiterhin der Bedarf nach einem Implantat, das nicht nur von Hartgewebezellen, wie Knochenzellen, sondern auch von Weichgewebezellen wie Gingivazellen angenommen wird. Es hat sich gezeigt, dass Weichgewebezellen andere Oberflächenstrukturen als Hartgewebezellen bevorzugen. Um zu vermeiden, dass es insbesondere an den Kontaktstellen zwischen Implantat und Zahnfleisch zu einem Zurückweichen des Zahnfleischs und damit zu einem Freiliegen zumindest eines Teils des Implantats kommt, muss daher eine Lösung gefunden werden, die die Anlagerung beider Zelltypen, Hartgewebezellen und Weichgewebezellen, an die Oberfläche des Implantats fördert.

Es ist daher Aufgabe der vorliegenden Erfindung ein Zahnimplantat zur Verfügung zu stellen, das nicht nur eine gute Verwachsung mit dem Knochen zeigt, sondern auch die Anlagerung von Weichgewebezellen, insbesondere Gingivazellen, erlaubt.

Diese Aufgabe wird durch ein Zahnimplantat gelöst, das verschiedene Bereiche mit unterschiedlicher Oberflächenrauheit aufweist.

Ein erster Gegenstand der vorliegenden Erfindung ist daher ein Zahnimplantat umfassend
a) einen enossalen Bereich zum Einwachsen in einen Kieferknochen;
b) ein Abutment zur Aufnahme einer prothetischen Versorgung; und
c) einen Implantathals zur Anlagerung von Gingivazellen, wobei der Implantathals zwischen dem enossalen Bereich und dem Abutment angeordnet ist, und wobei der enossale Bereich, der Implantathals und das Abutment jeweils voneinander unterschiedliche mittlere Oberflächenrauheiten Rₐ, gemessen gemäß DIN EN ISO 4287, aufweisen.

Durch die unterschiedlichen Oberflächenrauheiten des enossalen Bereichs, des Implantathalses und des Abutments wird eine verbesserte Anlagerung der unterschiedlichen Gewebezellen erreicht, wobei insbesondere durch eine entsprechende Oberflächenrauheit des Implantathalses vermieden wird, dass es zu einem Freiliegen des Implantathalses durch zurückweichendes Zahnfleisch kommt. Das erfindungsgemäße Zahnimplantat zeigt so eine hervorragende Einwachszeit, auch bei unterschiedlichen Gewebearten und bietet einen stabilen Verbund mit der prothetischen Versorgung. Durch die unterschiedlichen Oberflächenrauheiten der verschiedenen Bereiche des erfindungsgemäßen Zahnimplantats kann die Behandlungsdauer deutlich verkürzt und das Implantat bereits kurze Zeit nach dem Einsetzen in den Kieferknochen belastet werden, wodurch eine verbesserte Versorgung des Patienten erreicht wird. So beträgt beispielsweise die Einwachszeit der Implantate des Standes der Technik üblicherweise 3 Monate für den Unterkiefer und 6 Monate für den Oberkiefer während für die erfindungsgemäßen Implantate die Zeiten deutlich reduziert werden konnten. Für die erfindungsgemäßen Implantate beträgt die Einwachszeit für den Unterkiefer etwa 2 Monate und für den Oberkiefer etwa 4 Monate.

Um einen optimalen Halt des Implantats im Kieferknochen zu erreichen, hat es sich als vorteilhaft erwiesen, den enossalen Bereich des Implantats, also der Teil des Implantats, der in den Kieferknochen eingebracht wird und der Verankerung im Kieferknochen dient, in Form eines Gewindes auszubilden. Daher ist eine Ausführungsform der vorliegenden Erfindung bevorzugt, in der der enossale Bereich des Zahnimplantats ein Gewinde aufweist. Vorzugsweise handelt es sich bei dem Gewinde um ein selbstschneidendes Gewinde. Die Länge des Gewindes ist vorzugsweise so gewählt, dass eine ausreichende Verankerung im Knochen erzielt wird.

Im Rahmen der vorliegenden Erfindung hat es sich gezeigt, dass die Einwachsgeschwindigkeit des Implantats weiterhin verbessert werden kann, wenn die Oberfläche des enossalen Bereichs neben der im Stand der Technik vorgeschlagenen Makrorauheit eine diese überlagernde Mikrorauheit aufweist. Durch das Anrauen der Oberfläche wird zwar die Oberfläche, die den Knochenzellen für eine Anlagerung zur Verfügung steht, vergrößert, es wurde jedoch gefunden, dass übliche, beispielsweise mittels Sandbestrahlung angeraute Oberflächen scharfe Kanten aufweisen, die von Knochenzellen gemieden werden.

Im Rahmen der vorliegenden Erfindung konnte durch Abrunden dieser Kanten eine Oberfläche geschaffen werden, die neben einer makroskopischen Oberflächenrauheit eine diese überlagernde Mikrorauheit aufwies, was sich in einer gewissen Porosität der Oberfläche widerspiegelt. Es wurde überraschend gefunden, dass die Anlagerung der Knochenzellen signifikant verbessert werden kann, wenn die makrostrukturierte Oberfläche des enossalen Bereichs weiterhin eine Mikrorauheit aufwies. So konnte in klinischen Versuchen mit menschlichen CAL72 Osteosarcomazellen bereits nach 24 Stunden eine deutlich erhöhte Anlagerung der Zellen beobachtet werden, im Vergleich zu herkömmlichen Implantaten. Entsprechend ist eine Ausführungsform der vorliegenden Erfindung bevorzugt, in der die Oberfläche des enossalen Bereichs des erfindungsgemäßen Zahnimplantats eine die makrostrukturierte Oberfläche überlagernde Mikrorauheit aufweist. Diese Mikrorauheit äußerst sich insbesondere durch eine Porosität der makrostrukturierten Oberfläche und abgerundete Oberflächenstrukturen.

In einer bevorzugten Ausführungsform weist die Oberfläche des enossalen Bereich des erfindungsgemäßen Zahnimplantats eine Mikrostruktur mit einer mittleren Porengröße von 2 µm oder weniger, vorzugsweise 0,5 bis 1 µm auf. Die mittlere Porengröße der Makrostruktur liegt in etwa bei 4 bis 8 µm.

Eine entsprechend strukturierte Oberfläche ist beispielsweise durch Behandlung der Oberfläche mittels Sandstrahlen gefolgt von chemischem Ätzen der Oberfläche und einer abschließenden Wärmebehandlung erhältlich. Daher ist eine Ausführungsform bevorzugt, in der der enossale Bereich des erfindungsgemäßen Zahnimplantats durch Behandlung der entsprechenden Oberfläche mittels Sandstrahlen, gefolgt von chemischem Ätzen und einer abschließenden Wärmebehandlung erhalten wird. In einer bevorzugten Ausführungsform wird die Wärmebehandlung bei Temperaturen von 900 bis 1500 °C, vorzugsweise 1200 bis 1400 °C vorgenommen. Dabei wurde gefunden, dass durch die Behandlung der Oberfläche mittels Sandstrahlen deutlich verbesserte Oberflächenrauheiten erhalten werden können als beispielsweise mittels maschineller Bearbeitung der Oberfläche. Weiterhin wurde überraschend gefunden, dass die anschließenden Behandlungen der Oberfläche wie das chemische Ätzen und die Wärmebehandlung keinen Einfluss auf die eigentliche Makrostrukturierung der Oberfläche haben, sondern lediglich die gewünschte Mikrostrukturierung erlauben. Ein geeignetes Verfahren zum Erhalt einer solchen Oberfläche ist beispielsweise in WO 2009/007338 beschrieben.

In einer bevorzugten Ausführungsform des erfindungsgemäßen Zahnimplantats weist der enossale Bereich eine Makrorauheit mit Werten der mittleren Oberflächenrauheit Rₐ(Eₘₐₖᵣₒ) von 1,0 bis 2,0 µm, vorzugsweise 1,2 bis 1,8 µm, insbesondere von 1,4 bis 1,6 µm auf, bestimmt gemäß DIN EN ISO 4287. Es wurde überraschend gefunden, dass die Einwachszeit des Implantats deutlich verkürzt werden konnte, wenn der enossale Bereich eine mittlere Oberflächenrauheit im beanspruchten Bereich aufweist.

In einer bevorzugten Ausführungsform weist der enossale Bereich des erfindungsgemäßen Zahnimplantats eine Mikrorauheit mit Werten der mittleren Oberflächenrauheit Rₐ(Eₘᵢₖᵣₒ) von 0,1 bis 0,7 µm, vorzugsweise 0,2 bis 0,5 µm auf. Besonders bevorzugt beträgt das Verhältnis von Makrorauheit zu Mikrorauheit, ausgedrückt als Rₐ(Eₘₐₖᵣₒ)/Rₐ(Eₘᵢₖᵣₒ) des enossalen Bereichs 20:1 bis 3:1, vorzugsweise 10:1 bis 1,5:1.

Die Makrorauheit der Oberfläche kann beispielsweise mit Hilfe eines Hommel-Wave-Systems (Hommel Wave, VS-Schwenningen, Deutschland) bestimmt werden. Die Mikrostruktur einer solchen Oberfläche lässt sich beispielsweise mit konfokaler Laserscanningmikroskopie (LEXT OLS4000, Olympus, Tokyo, Japan) analysieren.

Das erfindungsgemäße Zahnimplantat ist so ausgebildet, dass eine optimale Verankerung im Knochen erreicht wird. Um Knochenverlust durch Druckbelastung, beispielsweise bei einem zu tief im Knochen sitzenden Implantat zu vermeiden, hat es sich als vorteilhaft erwiesen, wenn der maximale Durchmesser des Implantathalses den Durchmesser des enossalen Bereich des Implantats nicht übersteigt. Daher ist eine Ausführungsform des erfindungsgemäßen Zahnimplantats bevorzugt, in der das Zahnimplantat stiftförmig ausgebildet ist, wobei der maximale Durchmesser des Implantathalses kleiner oder gleich dem maximalen Durchmesser des enossalen Bereichs ist. Auf diese Weise kann ein guter vertikaler Knochenerhalt gewährleistet werden. Der maximale Durchmesser bezeichnet dabei jeweils eine durch die Mittelachse des Zahnimplantats verlaufende Gerade, die den größtmöglichen Abstand zweier Schnittpunkte mit einer das Zahnimplantat im entsprechenden Bereich orthogonal zur Mittelachse umlaufenden, dem Umfang des Zahnimplantats in diesem Bereich entsprechenden Linie bildet. Die Mittelachse des Zahnimplantats entspricht dabei der Achse, die in Richtung der größten Ausdehnung des Implantats durch dessen Mittelpunkt verläuft.

Der Implantathals ist in der Regel der Teil (Bereich) eines Zahnimplantats, der mit dem Weichgewebe, insbesondere dem Zahnfleisch, in Kontakt kommt und an den sich Gingivazellen anlagern sollen, um einen optimalen Halt und eine lange Nutzung des Implantats zu gewährleisten. Um eine Anlagerung der Gingivazellen zu fördern hat es sich als vorteilhaft erwiesen, wenn der Implantathals eine deutlich geringere Oberflächenrauheit als der enossale Bereich des Implantats aufweist. Eine optimale und anhaltende Anlagerung der Gingivazellen an den Implantathals konnte erreicht werden, wenn die mittlere Oberflächenrauheit des Implantathalses nicht mehr als 0,25 µm aufweist. Daher ist eine Ausführungsform der vorliegenden Erfindung bevorzugt, in der der Implantathals des erfindungsgemäßen Zahnimplantats eine mittlere Oberflächenrauheit Rₐ(H) von 0,04 bis 0,2 µm, vorzugsweise 0,06 bis 0,16 µm und besonders bevorzugt 0,08 bis 0,12 µm aufweist. In einer besonders bevorzugten Ausführungsform weist der Implantathals eine mittlere Oberflächenrauheit Rₐ(H) von weniger als 0,10 µm auf. Die mittlere Oberflächenrauheit wird dabei gemäß DIN EN ISO 4287 gemessen. Es hat sich im Rahmen der vorliegenden Erfindung gezeigt, dass durch Oberflächenrauheiten im beanspruchten Bereich zum einen die Anlagerung von Gingivazellen gefördert wird, zum anderen aber auch ein späteres Zurückweichen des Zahnfleischs an der Eindringstelle des Implantats in den Kiefer verhindert werden kann.

In einer besonders bevorzugten Ausführungsform weist das erfindungsgemäße Zahnimplantat ein Verhältnis der Oberflächenrauheiten des Implantathalses und des enossalen Bereichs (Rₐ(H)/Rₐ(E)) von 1:5 bis 1:50, vorzugsweise 1:10 bis 1:20 auf.

Um die Stabilität des erfindungsgemäßen Zahnimplantats zu verbessern, hat es sich als vorteilhaft erwiesen, wenn das Implantat zwischen dem enossalen Bereich und dem Implantathals, also dort wo der Übergang von Hartgewebe zu Weichgewebe erfolgt, eine Verjüngung aufweist. Daher ist eine Ausführungsform bevorzugt, in der der Implantathals einen konusförmigen Abschnitt aufweist, bei dem sich vorzugsweise der Durchmesser von der dem Abutment zugewandten Seite zu der dem enossalen Bereich zugewandten Seite verjüngt.

In einer besonders bevorzugten Ausführungsform weist der Implantathals einen konusförmigen Abschnitt und eine den Implantathals orthogonal zur Mittelachse des Zahnimplantats umlaufende Vertiefung auf, insbesondere eine horizontal umlaufende Rille auf. Es hat sich überraschend gezeigt, dass die Einbeziehung einer solchen Rille den Effekt des Plattformswitching fördert, wobei durch den geringeren Durchmesser im Vergleich zum enossalen Bereich des erfindungsgemäßen Zahnimplantats in diesem Bereich ein guter vertikaler Knochenerhalt beobachtet wurde. Die den Implantathals horizontal umlaufene Rille grenzt vorzugsweise direkt an den enossalen Bereich des Implantats an. In einer besonders bevorzugten Ausführungsform ist die Rille zwischen dem enossalen Bereich und dem konusförmigen Abschnitt des Implantathalses angeordnet. In einer besonders bevorzugten Ausführungsform hat diese Rille eine Breite von 0,1 bis 0,4 mm, vorzugsweise 0,15 bis 0,3 mm. Als weiterer Vorteil kann die Rille in dem erfindungsgemäßen Zahnimplantat dazu herangezogen werden, die ideale Einschraubtiefe des Implantats zu kennzeichnen.

Der konusförmige Abschnitt des Implantathalses weist vorzugsweise die gleiche mittlere Oberflächenrauheit Rₐ wie der Implantathals auf. In einer bevorzugten Ausführungsform weist der konusförmige Abschnitt des Implantathalses eine Höhe von 0,5 bis 3 mm, vorzugsweise 1,5 bis 2,5 mm auf.

Das erfindungsgemäße Zahnimplantat weist weiterhin ein Abutment zur Aufnahme einer prothetischen Versorgung auf. Bei der prothetischen Versorgung handelt es sich beispielsweise um eine Brücke oder eine Krone. Design und Oberfläche des Abutments sind für einen optimalen Verbund des Implantats mit einer prothetischen Versorgung gestaltet. In einer bevorzugten Ausführungsform weist das Abutment daher eine oder mehrere Vertiefungen auf, um einen sicheren Verbund mit der prothetischen Versorgung zu ermöglichen. Ein solche Vertiefung kann beispielsweise dazu verwendet werden, die prothetische Versorgung mittels Klammern oder nach dem Nut-und-Feder-Prinzip zu befestigen.

In einer besonders bevorzugten Ausführungsform weist das Abutment eine Lamellengestaltung auf. Bei diesen Lamellen handelt es sich vorzugsweise um eine oder mehrere horizontale Mikrorillen in der Oberfläche des Abutments. Diese besondere Ausgestaltung der Oberfläche des Abutments verbessert wesentlich die definitive Befestigung der prothetischen Versorgung.

In einer weiterhin bevorzugten Ausführungsform weist das Abutment des erfindungsgemäßen Zahnimplantats eine mittlere Oberflächenrauheit Rₐ(A) von 0,25 bis 0,7 µm, vorzugsweise 0,3 bis 0,6 µm, besonders bevorzugt 0,3 bis 0,5 µm auf, jeweils bestimmt gemäß DIN EN ISO 4287. In einer bevorzugten Ausführungsform weist das Abutment eine Höhe von 2,5 bis 5,5 mm, vorzugsweise 3,5 bis 5,0 mm auf. Es wurde überraschend gefunden, dass mit einem Abutment in der gewählten Höhe eine Winkelkorrektur im Frontzahnbereich beim Einpassen der prothetischen Versorgung vermieden werden kann.

In einer bevorzugten Ausführungsform weist das erfindungsgemäße Zahnimplantat ein Verhältnis der mittleren Oberflächenrauheiten des Implantathalses und des Abutments, ausgedrückt als Rₐ(H)/Rₐ(A) von 1:20 bis 1:1,5, vorzugsweise 1:10 bis 1:3,5 auf, wobei die Oberflächenrauheiten jeweils gemäß DIN EN ISO 4287 bestimmt sind.

In einer weiterhin bevorzugten Ausführungsform weist das erfindungsgemäße Zahnimplantat ein Verhältnis der mittleren Oberflächenrauheiten des Abutments und des enossalen Bereichs, ausgedrückt als Rₐ(A)/Rₐ(E) von 1:8 bis 1:1,5, vorzugsweise 1:5 bis 1:3,5 auf, wobei die Oberflächenrauheiten jeweils gemäß DIN EN ISO 4287 bestimmt sind.

Das erfindungsgemäße Zahnimplantat ist vorzugsweise einstückig ausgebildet. An das Material des Implantats sind keine besonderen Anforderungen zu stellen, solange es biokompatibel und gesundheitlich unbedenklich ist. In einer besonders bevorzugten Ausführungsform besteht das erfindungsgemäße Zahnimplantat ganz oder teilweise aus einem Material, das ausgewählt ist aus der Gruppe bestehend aus Metall, Keramik, Glaskeramik, Kunststoff und Kombinationen davon. Als besonders geeignete Materialien haben sich Oxidkeramiken herausgestellt. Daher ist eine Ausführungsform bevorzugt, in der das erfindungsgemäße Zahnimplantat ganz oder teilweise aus einer Oxidkeramik besteht, umfassend ein oder mehrere Oxide der Metalle ausgewählt aus Aluminium, Zirkonium, Yttrium, Cer, Hafnium, Magnesium und Mischungen und Kombinationen davon. In einer besonders bevorzugten Ausführungsform besteht oder umfasst das erfindungsgemäße Zahnimplantat ganz oder teilweise aus stabilisiertem Zirkoniumoxid. In einer insbesondere bevorzugten Ausführungsform wird als Material zur Herstellung des erfindungsgemäßen Zahnimplantats mit Yttriumoxid stabilisiertes Zirkoniumoxid verwendet, wobei der Anteil an mit Yttriumoxid stabilisiertem Zirkoniumoxid vorzugsweise mindestens 90 Gew.-%, besonders bevorzugt mindestens 95 Gew.-% und insbesondere mindestens 98 Gew.-% beträgt, jeweils bezogen auf das Gesamtgewicht des Zahnimplantats. Durch einen nachträglichen isostatischen Druckprozess wird die Materialqualität verbessert und senkt das Versagensrisiko des Implantats.

Das Material des erfindungsgemäßen Zahnimplantats ist vorzugsweise zahnfarben. Auf diese Weise kann die Grundlage für eine hohe Ästhetik der prothetischen Versorgung gelegt werden und ein "Durchschimmern" des Implantats wird verhindert.

Details und Vorteile der vorliegenden Erfindung werden anhand der folgenden Zeichnungen näher veranschaulicht, wobei diese keineswegs als Einschränkung des Erfindungsgedankens zu verstehen sind.

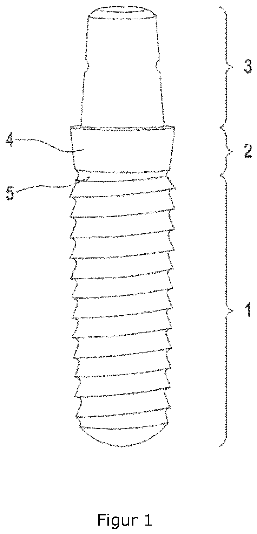

Figur 1 zeigt exemplarisch eine schematische Darstellung eines erfindungsgemäßen Zahnimplantats mit enossalem Bereich 1 (der Teil es Zahnimplantats, der der Verankerung im Kieferknoch dient), Abutment 3 und Implantathals 2. Weiterhin dargestellt wird ein konusförmiger Abschnitt 4, der den Implantathals 2 ausbildet und eine horizontal umlaufende Rille 5, die unterhalb des konusförmigen Abschnitts 4 angeordnet ist and die sich das Gewinde des enossalen Bereichs anschließt.

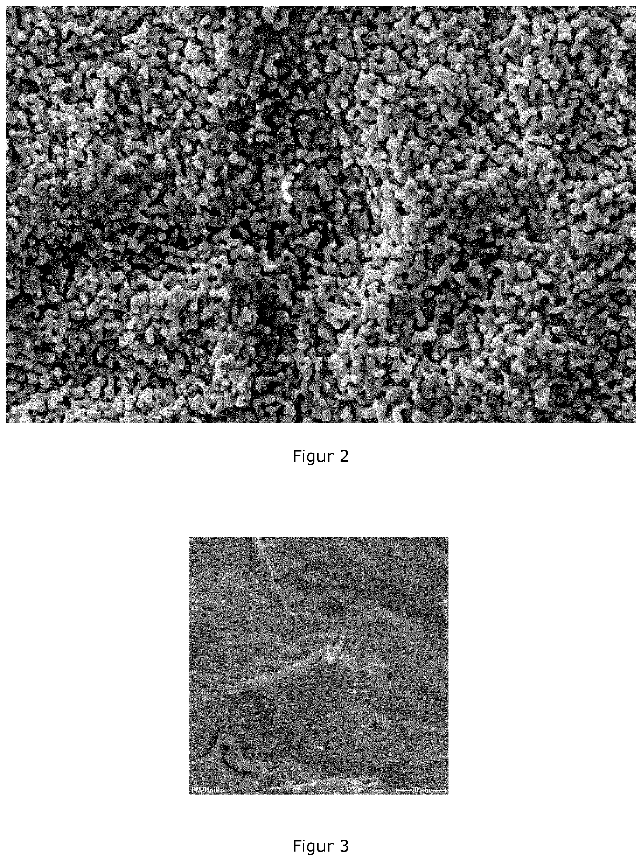

Figur 2 zeigt eine SEM-Aufnahme der Oberfläche des enossalen Bereichs des erfindungsgemäßen Zahnimplantats, wobei deutlich die Mikrostrukturierung der makroskopisch rauen Oberfläche zu erkennen ist. Die in der Aufnahme dunkler erscheinenden Bereiche liegen tiefer und bilden eine physikalische Retention des Implantats direkt nach dem Einbringen des Implantats in den Knochen. Ohne an eine bestimmte Theorie gebunden zu sein, wird angenommen, dass der beim Einbringen des Implantats verdrängte spongiöse Knochen diese Makrorauheit belegt und so die verbesserte Primärfestigkeit des erfindungsgemäßen Implantats bewirkt. Die beobachtete hohe Akzeptanz der Knochenzellen wird auf die Mikrorauheit der Oberfläche zurückgeführt und schafft die Voraussetzung für ein spaltfreies Einwachsen des erfindungsgemäßen Zahnimplantats.
Figur 3 zeigt auf der Oberfläche des enossalen Bereichs eines erfindungsgemäßen Zahnimplantats angelagerte Knochenzellen. Deutlich ist die flächige Anlagerung / Ausdehnung der Zellen zu erkennen.

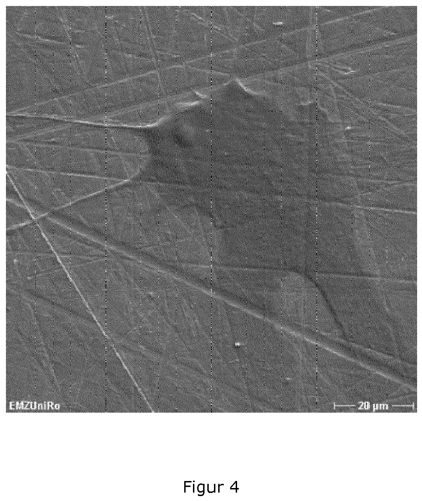

Figur 4 zeigt die Ausbreitung von Gingivafibroblasten nach 24 Stunden auf dem Implantathals eines erfindungsgemäßen Zahnimplantats.

## Patentansprüche

1. Zahnimplantat umfassend
einen enossalen Bereich (1) zum Einwachsen in einen Kieferknochen,
ein Abutment (3) zur Aufnahme einer prothetischen Versorgung, und einen zwischen dem enossalen Bereich (1) und dem Abutment (3) angeordneten Implantathals (2) zur Anlagerung von Gingivazellen, **dadurch gekennzeichnet, dass** der enossale Bereich (1), der Implantathals (2) und das Abutment (3) jeweils voneinander unterschiedliche mittlere Oberflächenrauheiten Rₐ, gemessen gemäß DIN EN ISO 4287, aufweisen.

2. Zahnimplantat gemäß Anspruch 1, **dadurch gekennzeichnet, dass** der enossale Bereich (1) ein Gewinde aufweist.

3. Zahnimplantat gemäß Anspruch 1 oder 2, **dadurch gekennzeichnet, dass** der enossale Bereich eine mittlere Oberflächenrauheit Rₐ(E) von 1,0 bis 2,0 µm, vorzugsweise von 1,2 bis 1,8 µm und insbesondere von 1,4 bis 1,6 µm, gemessen gemäß DIN EN ISO 4287 aufweist.

4. Zahnimplantat gemäß einem oder mehreren der vorangehenden Ansprüche, **dadurch gekennzeichnet, dass** das Zahnimplantat stiftförmig ausgebildet ist, wobei der maximale Durchmesser des Implantathalses (2) kleiner oder gleich dem maximalen Durchmesser des enossalen Bereichs (1) ist.

5. Zahnimplantat gemäß einem oder mehreren der vorangehenden Ansprüche, **dadurch gekennzeichnet, dass** der Implantathals (2) eine mittlere Oberflächenrauheit Rₐ(H) von 0,04 bis 0,2 µm, vorzugsweise von 0,06 bis 0,16 µm und insbesondere von 0,08 bis 0,12 µm oder bis 0,10 µm, gemessen gemäß DIN EN ISO 4287, aufweist.

6. Zahnimplantat gemäß einem oder mehreren der vorangehenden Ansprüche, **dadurch gekennzeichnet, dass** der Implantathals (2) einen konusförmigen Abschnitt (4) aufweist, bei dem sich vorzugsweise der Durchmesser von der dem Abutment (3) zugewandten Seite zu der dem enossalen Bereich (1) zugewandten Seite verjüngt.

7. Zahnimplantat gemäß einem oder mehreren der vorangehenden Ansprüche, **dadurch gekennzeichnet, dass** der Implantathals (2) einen konusförmigen Abschnitt (4) und eine horizontal umlaufende Vertiefung (5), insbesondere eine horizontal umlaufende Rille, aufweist.

8. Zahnimplantat gemäß einem oder mehreren der vorangehenden Ansprüche, **dadurch gekennzeichnet, dass** der Implantathals (2) eine horizontal umlaufende Rille (5) aufweist, die direkt an den enossalen Bereich (1) angrenzt.

9. Zahnimplantat gemäß Anspruch 7, **dadurch gekennzeichnet, dass** der Implantathals (2) eine horizontal umlaufende Rille (5) aufweist, die zwischen dem enossalen Bereich (1) und dem konusförmigen Abschnitt (4) des Implantathalses angeordnet ist.

10. Zahnimplantat gemäß einem oder mehreren der vorangehenden Ansprüche, **dadurch gekennzeichnet, dass** das Abutment (3) ein oder mehrere Vertiefungen aufweist.

11. Zahnimplantat gemäß einem oder mehreren der vorangehenden Ansprüche, **dadurch gekennzeichnet, dass** das Abutment (3) eine oder mehrere Mikrorille(n) aufweist.

12. Zahnimplantat gemäß einem oder mehreren der vorangehenden Ansprüche, **dadurch gekennzeichnet, dass** das Abutment (3) eine mittlere Oberflächenrauheit Rₐ(A) von 0,25 bis 0,7 µm, vorzugsweise von 0,3 bis 0,6 µm und insbesondere von 0,3 bis 0,5 µm, gemessen gemäß DIN EN ISO 4287, aufweist.

13. Zahnimplantat gemäß einem oder mehreren der vorangehenden Ansprüche, **dadurch gekennzeichnet, dass** das Zahnimplantat einstückig ausgebildet ist.

14. Zahnimplantat gemäß einem oder mehreren der vorangehenden Ansprüche, **dadurch gekennzeichnet, dass** das Zahnimplantat ganz oder teilweise aus einem Material besteht, das ausgewählt ist aus der Gruppe bestehend aus Metall, Keramik, Glaskeramik, Kunststoff und Kombinationen davon.

15. Zahnimplantat gemäß einem oder mehreren der vorangehenden Ansprüche, **dadurch gekennzeichnet, dass** das Zahnimplantat ganz oder teilweise aus einer Oxidkeramik besteht, umfassend ein oder mehrere Oxide der Metalle ausgewählt aus Aluminium, Zirkonium, Yttrium, Cer, Hafnium und Magnesium.
